# EUROPEAN PATENT APPLICATION

(11) **EP 3 919 609 A1**
(43) Date of publication of application: **08.12.2021**
(21) Application number: 21177181.1
(22) Date of filing: 01.06.2021
(51) Int. Cl.: C12M 1/32, C12M 3/00, C12M 1/00

(54) **CELL CULTURE DISH**

(30) Priority: 02.06.2020 CN 202020987204 U
(71) Applicant: Shenzhen Vitavitro Biotech Co., Ltd., Shenzhen, Guangdong 518000 (CN)
(72) Inventor: LIN, Xiaozhen, 518000 Shenzhen (CN)
(74) Representative: Walaski, Jan Filip

(57) **Abstract**

A cell culture dish, comprising a dish bottom (11) , as well as an inner side wall (13) and an outer side wall (12) integrated with the dish bottom, wherein the inner part enclosed by the inner side wall forms a culture area, and a water tank (15) is formed between the inner side wall and the outer side wall, and the water tank is located on the periphery of the culture area (14); at least one convex ring (140) is provided on the surface of the dish bottom in the culture area, and the inner part enclosed by the convex ring forms a culture tank, wherein a number of culture microwells are provided at the bottom of at least one culture tank.

## Description

### Technical Field

The present utility model belongs to the technical field of cell culture, and relates to a cell culture dish.

### Background Art

In vitro culture of embryos is a key step in the field of assisted reproduction, and high-quality embryos affect subsequent implantation or pregnancy directly. Traditional incubators are large in size, and with the increase in the number of infertile patients, the demand for incubators also increases, but the space of the reproductive center laboratory is limited and cannot accommodate too many incubators. The volume of a desktop incubator is relatively small, while most of the desktop incubators are dry culture systems, which would lead to a risk of evaporation of culture medium.

In traditional culture process, embryos must be removed from the incubator for evaluation, and will be exposed to unnecessary pressure and fluctuations. At present, most embryo culture dishes used in clinic are ordinary flat-bottomed dishes, and they must be removed from the incubator when observing the embryos, which will affect the development of embryos. In addition, in current dishes, if the humidity of the surrounding environment is too low in the culture process, it will cause the culture medium in the culture dish to evaporate faster, and will easily cause the risk of osmotic pressure increase.

### Summary of the Present Utility Model

The objective of the present utility model is to provide a cell culture dish, which can be applied to the in vitro culture of human embryonic cells, and the culture dish can be matched with a time-lapse photographic apparatus, combining the advantages of culturing multiple cells together while keeping them in appropriate positions to be monitored and photographed separately, and the cells can be observed and evaluated in real time without disturbing the process of cell culture, to solve the problem of evaporation of culture medium in dry incubators, and to provide a saturated humidity environment for cell culture.

In order to achieve the above objective, the technical solution adopted by the present utility model is as follows.

The technical solution is a cell culture, comprising a dish bottom, as well as an inner side wall and an outer side wall integrated with the dish bottom, wherein the inner part enclosed by the inner side wall forms a culture area, and a water tank is formed between the inner side wall and the outer side wall, and the water tank is located on the periphery of the culture area; at least one convex ring is provided on the surface of the dish bottom in the culture area, and the inner part enclosed by the convex ring forms a culture tank, wherein a number of culture microwells are provided at the bottom of at least one culture tank.

The technical effect of the technical solution is that, ultra-pure water is added to the water tank, and the evaporation of water creates a humid culture environment around the culture area, to prevent the risk of osmotic pressure increase caused by the evaporation of culture medium; convex rings are provided in the culture area, wherein culture medium is added to the culture tank formed by the convex ring, to make the droplets of the culture medium relatively fixed, and not easy to move to cause the loss of embryos or the connection of droplets; culture microwells are provided in the culture tank, to make the location of the cells limited in the microwells during the culture process, and the whole process could be observed or monitored with photographic apparatus, and cells of best quality could be selected out without disturbing the process of cell culture.

In one example of the technical solutions, the height of the inner side wall is less than that of the outer side wall.

In one example of the technical solutions, the said water tank formed by the inner and outer side wall can hold water with a capacity of 0-15 ml.

In one example of the technical solutions, all the culture microwells in one culture tank are arranged in a matrix, and on the left side of the matrix, each row of the matrix is provided with a number or letter number at the front, and on the upper side of matrix, each column of the matrix is provided with a number or letter number on the top, wherein the letter number and the number form a two-dimensional positioning for each culture microwell, and each culture microwell is used to limit the cell during culture process.

In one example of the technical solutions, wave-shaped hand-held part(s) is provided on the external side of the outer side wall.

In one example of the technical solutions, the culture area is provided with a first culture tank and a first culture microwell in the first culture tank, and the first culture microwell includes an upper inverted rounded frustum and a lower cone, wherein the inclination of the side of the inverted rounded frustum from the vertical direction is 2-8 degrees, and the angle between the side of the cone and the horizontal direction is 4-10 degrees.

In one example of the technical solutions, the culture area is provided with a second culture tank and a second culture microwell in the second culture tank, and the bottom and the inside of the second culture microwell are both concave arcs.

In one example of the technical solutions, the culture area is provided with a third culture tank and a third culture microwell in the third culture tank, and the third culture microwell is the hole of an inverted rounded frustum.

In one example of the technical solutions, the culture area is provided with at least one spare tank enclosed and formed by a convex ring, and the spare tank is used for temporarily placing cells or for washing cells.

### Brief Description of the Figures

In order to explain the technical solutions in the embodiments of the present utility model more clearly, the figures that need to be used in the description of the embodiments of the present utility model will be briefly introduced below, and the figures in the following description are only for some embodiments, and for the person skilled in the art, other figures can be obtained based on these figures without creative work.
FIG. 1 shows the structural view of the culture dish in the embodiments of the present application.
FIG. 2 shows the front view of the structure of the culture dish in the embodiments of the present application.
FIG. 3 shows the structural view of the first culture tank in Fig. 2.
FIG. 4 shows the sectional view taken along A-A of the culture dish in Fig. 2.
FIG. 5 shows an enlarged view of part I in Fig. 4.
FIG. 6 shows the sectional view taken along B-B of the culture dish in Fig. 2.
FIG. 7 shows an enlarged view of Part II in Fig. 6.
FIG. 8 shows an enlarged view of Part III in Fig. 6.

### Detailed Description of Embodiments

The embodiments of the present utility model are described in detail below and examples of the embodiments are shown in the figures, in which all the same or similar reference numerals indicate the same or similar elements or elements with the same or similar functions. The embodiments described below with reference to the figures are exemplary, and are intended to explain the present utility model but should not be understood as a limitation to the present utility model.

In the description of the present utility model, it should be understood that the orientation or position relationship indicated by the terms "center", "longitudinal direction", "transverse direction", "length", "width", "thickness", "upper", "lower", "front", "back", "left", "right", "vertical", "horizontal", "top", "bottom", "inner", "outer", "clockwise", "anticlockwise" and so forth are based on the orientation or position relationship shown in the figures, and it is only for the convenience of describing the present utility model and simplifying the description, rather than indicating or implying that the device or element referred to must have a specific orientation or be constructed and operated in a specific orientation, so it cannot be understood as a limitation to the present utility model.

In addition, the terms "first" and "second" are only used for descriptive purpose, and cannot be understood as indicating or implying relative importance or implicitly indicating the number of the indicated technical features. Thus, the features defined with "first" and "second" can explicitly or implicitly include one or more of these features. In the description of the present utility model, "multiple" means two or more, unless otherwise specifically defined.

In the present utility model, unless otherwise explicitly specified or defined, the terms "installation", "connected", "connection", "fixed" and so forth shall be understood in a broad sense, and for example, it can be fixed connection, or detachable connection, or integrated connection; it can be mechanical connection, or electrical connection; it can be direct connection, or indirect connection through intermediate medium, and it can be internal connection of two elements. For the person skilled in the art, the specific meaning of the above-mentioned terms in the present utility model can be understood according to specific situations.

In the present utility model, unless otherwise explicitly specified or defined, the first feature "on" or "under" the second can include the first and second features in direct contact, and it can also include that the first and second features are not in direct contact but contact through other features between them. Moreover, the first feature "above", "up" or "on" the second feature includes the first feature being directly above or obliquely above the second feature, or only indicates that the horizontal level of the first feature is higher than that of the second feature. The first feature "below", "under" or "underneath" the second feature includes the first feature being directly below or obliquely below the second feature, or only indicates that the horizontal level of the first feature is less than that of the second feature.

In addition, the technical solutions of the various embodiments can be combined with each other, but it must be based on what can be achieved by the person skilled in the art, and when the combination of technical solutions is contradictory or cannot be achieved, it should be considered that such a combination of technical solutions does not exist and is not within the scope of the disclosure of the present utility model.

In the following description, the use of terms such as "module", "part", "component", or "unit" is only to facilitate the description of the present utility model, and they have no specific meanings in themselves. So they can be used in a mixed manner.

Hereinafter, the present utility model is further described in detail by specific embodiments along with the figures.

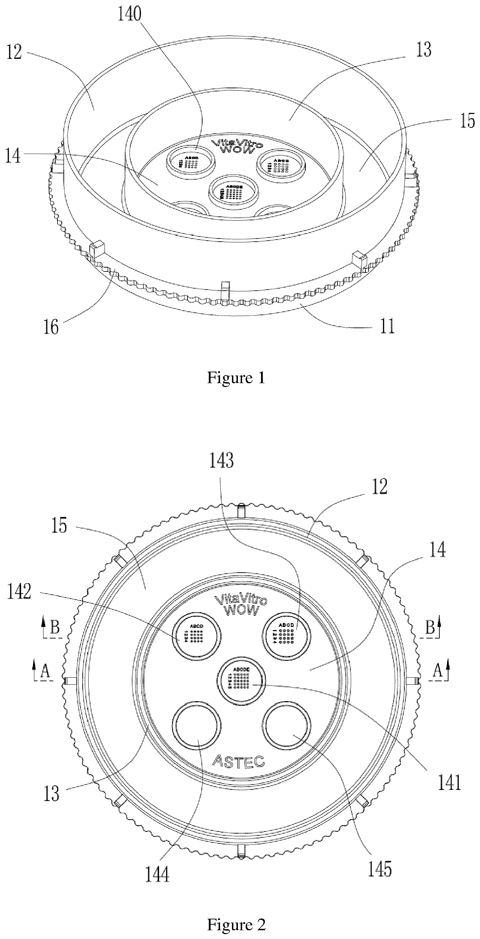

As shown in FIGS. 1, 2 and 4, the present embodiment is a culture dish for in vitro culture of human embryos, which is used for the culture of embryonic cells in assisted reproduction laboratories. The culture dish includes a rounded dish bottom 11, an outer side wall 12 and an inner side wall 13, wherein the dish bottom, the outer side wall and the inner side wall are an integrated structure, and the inner part enclosed by the inner side wall forms a culture area 14, and a water tank 15 is formed between the inner side wall and the outer side wall, and the water tank is located on the periphery of the culture area. Ultra-pure water is added to the water tank during culture process, to provide a humid environment for culture of the embryo cell(s) in the culture area; wave-shaped hand-held part(s) 16 is provided on the external side of the outer side wall for easy taking.

Five convex rings 140 are provided on the surface of the dish bottom in the culture area, and the inner parts enclosed by three convex rings form three culture tanks, which are the first culture tank 141, the second culture tank 142 and the third culture tank 143 respectively, and a number of culture microwells are provided at the bottom of each culture tank; the inner parts enclosed by two convex rings form two spare tanks, which are the first spare tank 144 used for temporarily placing embryos and the second spare tank 145 used for washing embryos.

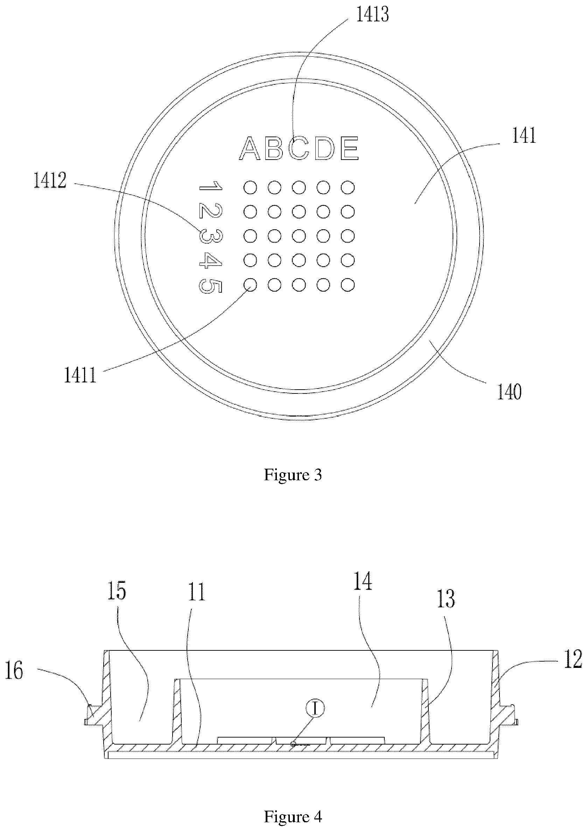

As shown in FIGS. 3 to 5, there are 25 first culture microwells 1411 provided in a 5 ^{∗} 5 matrix in the first culture tank, and the matrix composed of all the first culture microwells is located in the center of the convex ring, and the distance between adjacent first culture microwells is 0.25 mm. On the left side of the matrix, each row of the matrix is provided with a number 1412, and the numbers are "1, 2, 3, 4, 5"; On the upper side of the matrix, each column of the matrix is provided with a letter number 1413, and the letter numbers are "A, B, C, D, E"; based on the numbers and letter numbers, each first culture microwell can be positioned in two dimensions. For example, the first microwell in the upper left corner can be positioned with A1, and the first microwell in the lower right corner can be positioned with D5.

The first culture microwell 1411 is concave hole structure, including an upper inverted rounded frustum and a lower cone, wherein the inclination of the side of the inverted rounded frustum from the vertical direction is 5 degrees, and the angle between the side of the cone and the horizontal direction is 7 degrees, and the upper circle diameter of the inverted rounded frustum of the first culture microwell is 0.3 mm, the lower circle diameter is 0.28 mm, and the depth of the first culture microwell is 0.16 mm.

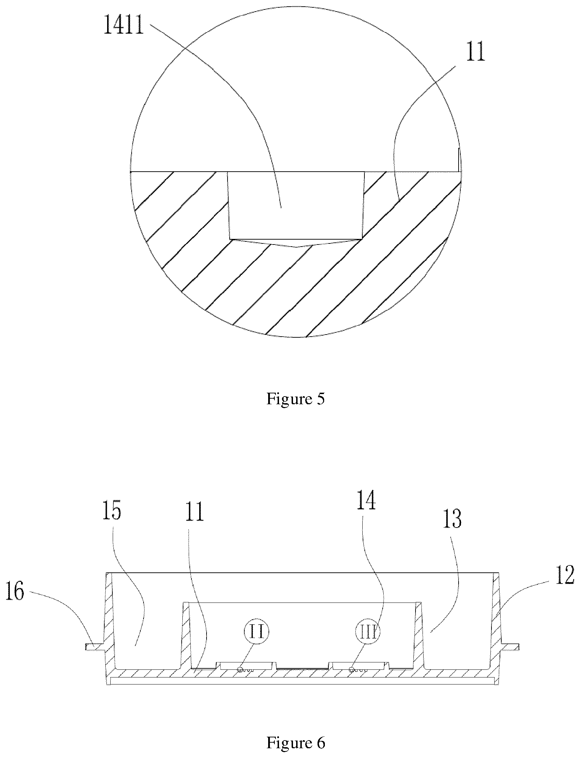

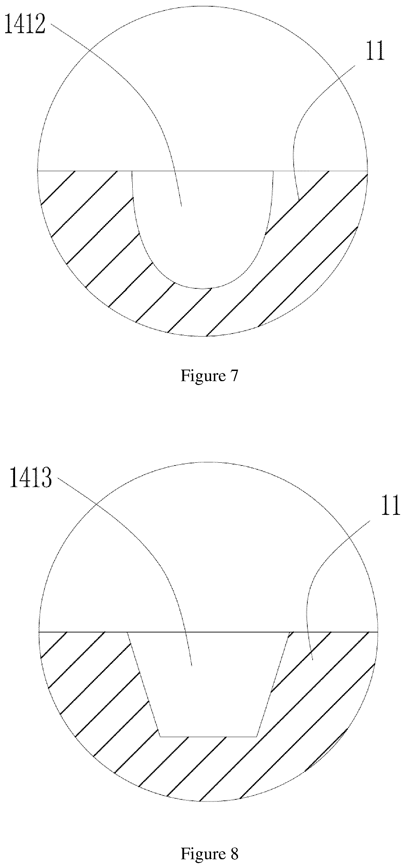

As shown in FIGS. 6 and 7, there are 16 second culture microwells 1421 provided in a 4 ^{∗} 4 matrix in the second culture tank 142, and all the second culture microwells have numbers and letter numbers of the same rules as the first culture microwells, and the bottom and the inside of the second culture microwells are concave arcs.

As shown in FIGS. 6 and 8, there are 16 third culture microwells 1431 provided in a 4 ^{∗} 4 matrix in the third culture tank 143, and all the third culture microwells have numbers and letter numbers of the same rules as the first culture microwells, and the third culture microwells are holes of an inverted rounded frustum.

When using the culture dish of the present embodiment for embryo culture, ultra-pure water is added to the water tank, and the evaporation of water creates a humid culture environment around the culture area, which solves the problem of the evaporation of culture medium in dry incubators, and prevents the risk of osmotic pressure increase caused by the evaporation of culture medium; convex rings are provided in the culture area, wherein culture medium is added to the culture tank formed by the convex ring, to make the droplets of the culture medium relatively fixed, and not easy to move to cause the loss of embryos or the connection of droplets; culture microwells are provided in the culture tank, to make the location of the embryos limited in the microwells during the culture process, and the whole process could be observed or monitored with photographic apparatus, and embryos of best quality could be selected out without disturbing the process of embryo culture.

In the description of the specification, the reference terms, "one mode of implementation", "some modes of implementation", "one embodiment", "some embodiments", "one example", "specific examples", or "some examples", etc., mean that the specific features, structures, materials or characteristics described with the embodiment or example are included in at least one embodiment or example of the present utility model. In the present specification, the schematic expression of the above-mentioned terms does not necessarily refer to the same embodiment or example. Moreover, the specific features, structures, materials or characteristics described can be combined in an appropriate manner in any one or more embodiments or examples.

## Claims

1. A cell culture dish, comprising a dish bottom, as well as an inner side wall and an outer side wall integrated with the dish bottom, wherein the inner part enclosed by the inner side wall forms a culture area, and a water tank is formed between the inner side wall and the outer side wall, and the water tank is located on the periphery of the culture area; at least one convex ring is provided on the surface of the dish bottom in the culture area, and the inner part enclosed by the convex ring forms a culture tank, wherein a number of culture microwells are provided at the bottom of at least one culture tank.

2. The cell culture dish according to claim 1, wherein the height of the inner side wall is less than that of the outer side wall.

3. The cell culture dish according to claim 1, wherein the said water tank formed by the inner and outer side wall can hold water with a capacity of 0-15 ml.

4. The cell culture dish according to claim 1, wherein all the culture microwells in one culture tank are arranged in a matrix, and on the left side of the matrix, each row of the matrix is provided with a number or letter number at the front, and on the upper side of the matrix, each column of the matrix is provided with a number or letter number on the top, wherein the letter number and the number form a two-dimensional positioning for each culture microwell, and each culture microwell is used to limit the embryonic cell(s) during culture process.

5. The cell culture dish according to any one of claims 1 to 4, wherein wave-shaped hand-held part(s) is provided on the external side of the outer side wall.

6. The cell culture dish according to any one of claims 1 to 4, wherein the culture area is provided with a first culture tank and a first culture microwell in the first culture tank, and the first culture microwell includes an upper inverted rounded frustum and a lower cone, wherein the inclination of the side of the inverted rounded frustum from the vertical direction is 2-8 degrees, and the angle between the side of the cone and the horizontal direction is 4-10 degrees.

7. The cell culture dish according to any one of claims 1 to 4, wherein the culture area is provided with a second culture tank and a second culture microwell in the second culture tank, and the bottom and the inside of the second culture microwell are concave arcs.

8. The cell culture dish according to any one of claims 1 to 4, wherein the culture area is provided with a third culture tank and a third culture microwell in the third culture tank, and the third culture microwell is the hole of an inverted rounded frustum.

9. The cell culture dish according to any one of claims 1 to 4, wherein the culture area is provided with at least one spare tank enclosed and formed by a convex ring, and the spare tank is used for temporarily placing cells or for washing cells.
